# EUROPEAN PATENT APPLICATION

(11) **EP 4 744 658 A1**
(43) Date of publication of application: **20.05.2026**
(21) Application number: 25215879.5
(22) Date of filing: 14.11.2025
(51) Int. Cl.: A61K 31/405, A61K 36/88, A61P 13/08, A23L 33/105, A61K 36/899, A61P 35/00, A61K 36/752

(54) **MULTICOMPONENT BOTANICAL COMPOSITION AND ITS USE IN THE TREATMENT OF PROSTATE DISORDERS**

(30) Priority: 14.11.2024 IT 202400025683
(71) Applicant: Kolinpharma S.p.A., 20122 Milano (IT)
(72) Inventor: PETRELLI, Rita Paola, 20122 Milano (IT); CURTI, Valeria, 20122 Milano (IT); COZZI, Marco, 20122 Milano (IT); BATTAGLIA, Stefania, 20122 Milano (IT); RANIERI, Francesca Romana, 20122 Milano (IT)
(74) Representative: Hoffmann Eitle

(57) **Abstract**

The present invention relates to mixtures, compositions comprising said mixtures, and their uses in therapy, since they are capable of treating, in a preventive and/or curative way, prostate pathologies and/or symptoms, preferably non-bacterial prostatitis, chronic pelvic pain syndrome (CP/CPPS) and/or benign prostate hyperplasia (BPH). In more detail, the present invention relates to a mixture comprising or, alternatively, consisting of: a) a *Secale cereale* (L.) pollen extract, preferably a pollen extract of the type of the commercial product Graminex^{®}, b) a quercetin, c) a bergamot extract, and d) a tryptophan, and/or a saffron extract.

## Description

The present invention relates to mixtures, compositions comprising said mixtures, and their uses in therapy, since they are capable of treating, in a preventive and/or curative way, prostate pathologies and/or symptoms, preferably non-bacterial prostatitis, chronic pelvic pain syndrome (CP/CPPS) and/or benign prostate hyperplasia (BPH).

In greater detail, the present invention relates to a mixture comprising or, alternatively, consisting of:
a) a *Secale cereale* (L.) pollen extract; and
b) a quercetin, and
c) a bergamot extract, and
d) a tryptophan and/or a saffron extract.

### Background of the invention

It is known that the prostate is a glandular organ of the male genital system that extends around the initial portion of the urethra and has two ejaculatory ducts passing through it. The prostate is cone-shaped and is situated between the medial margins of the two levator ani muscles.

Prostate-related diseases or symptoms are a public health problem with a considerable impact from an epidemiological, clinical and economic standpoint. From 35% to 50% of men report having been affected by symptoms attributable to prostatitis during their lifetime.

According to the National Institutes of Health (NIH), prostatitis are divided into four categories. The first (and less frequent) comprises acute bacterial prostatitis, characterised by the presence of bacteria (mainly Gram-negative, for example *Escherichia coli)* and acute infection of the prostate gland. The second category comprises chronic bacterial prostatitis (mainly Gram-negative, for example *Escherichia coli).*

These are chronic (persisting for at least three months) or recurrent infections of the prostate with a bacterial aetiology, characterised by painful urinary and sexual symptoms.

The third category comprises chronic non-bacterial prostatitis / chronic pelvic pain syndrome (CP/CPPS) and is the most common category of prostatitis among adult males. This third class can be further divided into inflammatory (Illa) and non-inflammatory (IIIb). Clinical symptoms and manifestations include pelvic or perineal pain (lower abdomen, testicles, penis) in the absence of pathogenic bacteria in the prostate secretions and difficulty in emptying (including irritative or obstructive symptoms). A very important aspect of this type of disorder is a reduction in the quality of life, which can lead to depression. The aetiology is still unknown and it would appear that infections are not the cause. Potential causes could be the presence of inflammations due to traumas, autoimmunity, a reaction to normal prostatic flora, neuropathic pain, an increase in pressure of the prostate tissue and interaction of somatic and psychological factors. Psychological stress, including anxiety and the fear of serious illnesses, would appear to be common in men with CP/CPPS symptoms and can be a factor that contributes to the onset and development of the pathology. Insofar as concerns treatment, a series of drugs and non-pharmacological therapies are available, including physical therapy and psychological support. There is no uniformly accepted treatment regime and many treatments are used in combination. The pharmacological therapies include: alpha-blockers, antibiotics, anti-inflammatory drugs, 5-alpha-reductase inhibitors and drugs for neuropathic pain.

Asymptomatic inflammatory prostatitis is in the fourth category and does not require pharmacological treatment.

Furthermore, an important disease of the prostate is benign prostatic hyperplasia (BPH). Benign prostatic hyperplasia (BPH) is a chronic condition associated with lower urinary tract symptoms (LUTS) and affects nearly 3 out of 4 men during the seventh decade of life. The mechanism that leads to the onset of BPH is not entirely clear, but various hypotheses have been advanced that involve metabolic and hormonal aspects and inflammatory processes. Systemic and localised inflammation, and also chronic inflammation, are associated with LUTS / BPH. Insofar as concerns the metabolic syndrome, scientific evidence highlights how this is correlated to lower urinary tract symptoms (LUTS). Metabolic syndrome means a condition characterised by the co-presence of: obesity, with fatty deposits at abdominal level (waistline in men > 102 cm), hypertriglyceridemia (> 150 mg/dl), HDL cholesterol levels lower than 40 mg/dl (in men), arterial hypertension (values higher than 130/85 mmHg) and high levels of fasting glycaemia (> 110 mg/dl). It has been demonstrated that patients with one or more of these symptoms show a more rapid progression to BHP, for example high levels of fasting insulin. At an anatomical level, the increase in size of the prostate brings about a compression of the urethra, causing a greater resistance in the urinary flow at the bladder outlet. The most widely used assessment scale of the symptoms is the IPSS, which is based on the classification of seven symptoms (weak urine stream, hesitancy, intermittency, feeling of incomplete emptying, difficulty in urinating, urgency, frequency, nocturia). The pharmacological treatment for BPH provides for an initial monotherapy based on alpha-1-adrenergic receptor antagonists, 5-alpha-reductase inhibitors, anticholinergic agents and phosphodiesterase 5 inhibitors, and, in the most serious cases, a combination thereof.

The most representative problems among those cited here above, and those for which management of the patient is more complex, are chronic non-bacterial prostatitis/chronic pelvic pain syndrome (CP/CPPS) and benign prostatic hyperplasia (BHP). In both cases, the patients complain of lower urinary tract symptoms (LUTS). Inflammation of the prostate is considered to be an important factor in determining both prostate enlargement and progression of the symptoms. The common aspects of inflammation are due to the activation of astrocytes and microglia, from involvement of the immune system, with the consequent hyper-expression of pro-inflammatory cytokines, in addition to an increase in reactive oxygen species and reactive nitrogen species (ROS/RNS).

It is known that neuro-inflammation is a key component of the pathogenesis of prostate diseases. In fact, inflammation of the prostate gland, present in the same way in both prostatitis and benign prostatic hyperplasia, also extends to the level of the neural tissue that surrounds the prostate, causing one of the most difficult symptoms to treat, which is neuropathic pain in the pelvic area.

Clinical observations suggest that chronic inflammation correlates with CP / CPPS and BPH. Furthermore, lowering of the quality of life of these patients lead to manifestation of states of depression.

Therefore, in order to treat these prostate pathologies and symptoms, preferably non-bacterial prostatitis, chronic pelvic pain syndrome (CP/CPPS) and benign prostatic hyperplasia (BHP), it is necessary to be able to have new therapeutic solutions capable of inhibiting inflammation and oxidative stress, and also acting at the level of mood, determining an improvement thereof.

US2023381263 (A1) describes compositions comprising a *Secale cereale* (L.) pollen extract, quercetin, lipoic acid and tryptophan and/or a saffron extract, for use in the treatment of prostate diseases and symptoms, in particular non-bacterial prostatitis/chronic pelvic pain syndrome (CP/CPPS) and/or benign prostatic hyperplasia (BHP). The technical problem that the present invention addresses and solves is to provide compositions that are effective and devoid of side effects for use in a treatment method, preventive and/or curative, of prostate pathologies and symptoms, preferably non-bacterial prostatitis, chronic pelvic pain syndrome (CP/CPPS) and/or benign prostatic hyperplasia (BHP).

Furthermore, the applicant addresses and solves the further problem of providing compositions for the treatment of prostate pathologies and symptoms which, in addition to being more effective than the compositions currently available, are also capable of successfully treating neuropathic pain in the pelvic area, considering that the neuro-inflammation that characterises prostate diseases also extends to the level of the neural tissue that surrounds the prostate, causing neuropathic pain in the pelvic area. In order to overcome said technical problems, the applicant, following an intensive phase of research and development, provides multicomponent mixtures or compositions (mixtures or compositions of the invention) comprising (a) a *Secale cereale* (L.) pollen extract (i.e., preferably, the commercial product Graminex^{®} G63^{®}) (hereinafter, briefly the "pollen extract"), (b) a quercetin, (c) a bergamot extract, and (d) a tryptophan and/or a saffron extract having a mood modulation activity, according to what is indicated in this description and in the accompanying claims.

Said mixtures or compositions of the invention, when administered to a subject in a state of need, are capable of preventing and/or treating prostate pathologies and symptoms, preferably non-bacterial prostatitis/chronic pelvic pain syndrome (CP/CPPS) and/or benign prostatic hyperplasia (BHP), in an effective and rapid way, and are also devoid of side effects.

Said treatment activity, preventive or curative, of the mixtures or compositions of the invention, is due to the specific and innovative combination of the active components (as defined in the present description), since each component acts with a different and complementary mechanism of action (immunostimulatory, anti-inflammatory, antioxidant and/or mood modulation), making the action of the composition of the invention particularly effective.

The mixtures and compositions of the invention, as they demonstrate a high safety profile, can be used by a broad category of subjects, such as adults, seniors and sports people. The mixtures and compositions of the invention are easy to prepare and economically advantageous.

These aims, and still others which will become clear from the detailed description that follows, are achieved by the compositions and mixtures of the present invention, thanks to the technical features present in the description and in the appended claims.

### Summary of the invention

A first aspect of the present invention relates to a mixture (briefly, mixture of the invention) comprising (a) a *Secale cereale* (L.) pollen extract, (b) a quercetin, (c) a bergamot extract, and (d) a tryptophan and/or a compound having a mood modulation activity similar to the tryptophan (for example, a saffron extract).

As demonstrated in the experimental part, the mixture of the invention is capable of acting on neuro-inflammation, increasing the level of the markers involved in the processes of protection of the myelin sheath and formation of neurites.

A second aspect of the present invention relates to a composition (briefly, composition of the invention) comprising said mixture, according to said first aspect of the invention, and at least one acceptable pharmaceutical or food grade additive and/or excipient.

A third aspect of the present invention relates to said mixture or composition of the invention for use as a medicament.

A fourth aspect of the present invention relates to said mixture or composition of the invention for use in a treatment method, preventive and/or curative, of prostate pathologies and symptoms, preferably non-bacterial prostatitis/chronic pelvic pain syndrome (CP/CPPS) and/or benign prostatic hyperplasia (BHP), in an individual having need, through the administration of a therapeutically effective amount of the mixture or composition of the invention to said individual.

Preferably, the mixtures and compositions that comprise said mixtures do not contain or include lipoic acid or its derivatives or salts.

Preferably, lipoic acid is understood to include a lipoic acid, both in oxidated and reduced form, and/or a pharmaceutically acceptable salt thereof and/or their mixtures.

### Description of the figures

In the Figures appended to this patent application, the terms "composition" and "combination" are used interchangeably and indicate the mixtures compared in the experimental part.

The terms BPF, TRP, POLL, QUE indicate the individual active ingredients, indicated here below:
BPF: Bergamot
TRP: L-tryptophan
POLL: rye pollen
QUE: Quercetin.
GGF: *glial growth factor* (negative control).

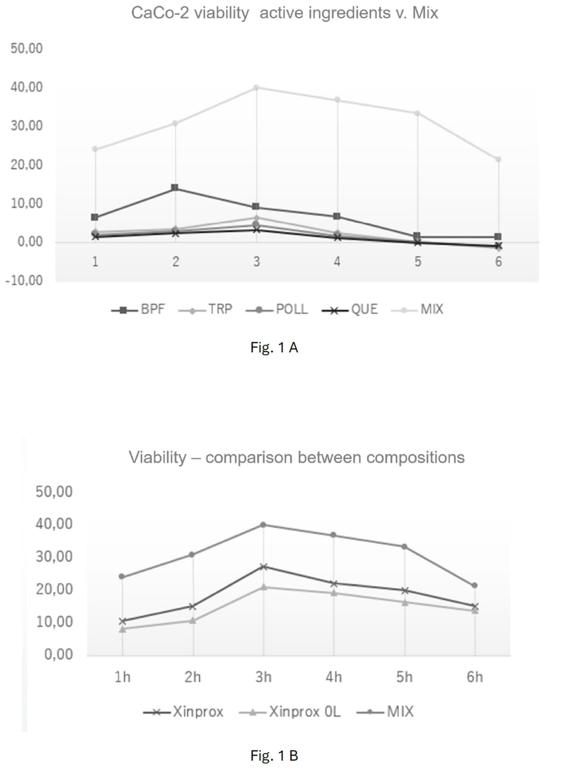

Figures 1 to 5 show the results of Phase 1 of the experiment described in the experimental part.

Figure 1 shows the viability of the mixture compared to A) the individual active ingredients and B) compared to the comparison compositions indicated in the experimental part. The MIX (mixture according to the present invention) is demonstrated to be statistically superior compared to the combination without lipoic acid and to the XINPROX formula (p<0.05 at all time points).

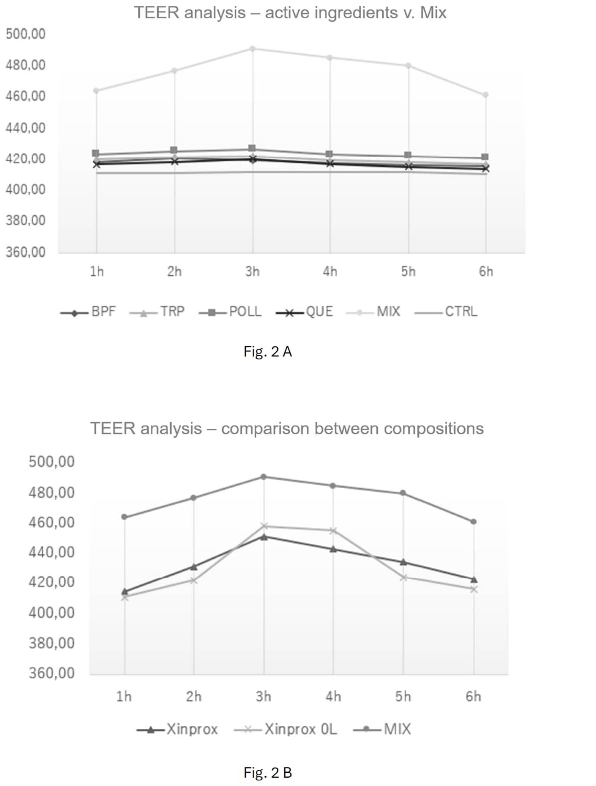

Figure 2 shows the TEER analysis of the mixture compared to A) the individual active ingredients and B) compared to the comparison compositions indicated in the experimental part. The mix is demonstrated to be statistically superior p<0.05 compared to the other two mixtures.

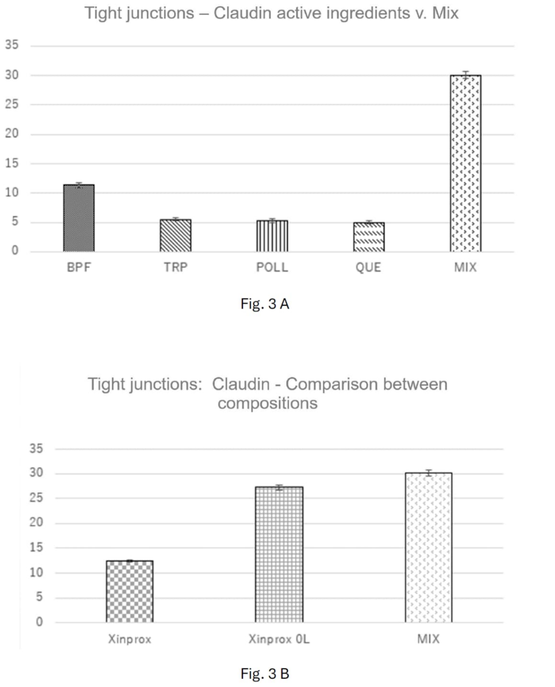

Figure 3 shows the integrity of the tight junctions, in particular of Claudin, in the mixture compared to A) the individual active ingredients and B) compared to the comparison compositions indicated in the experimental part. The mix is demonstrated to be statistically superior p<0.05 compared to the other two mixtures.

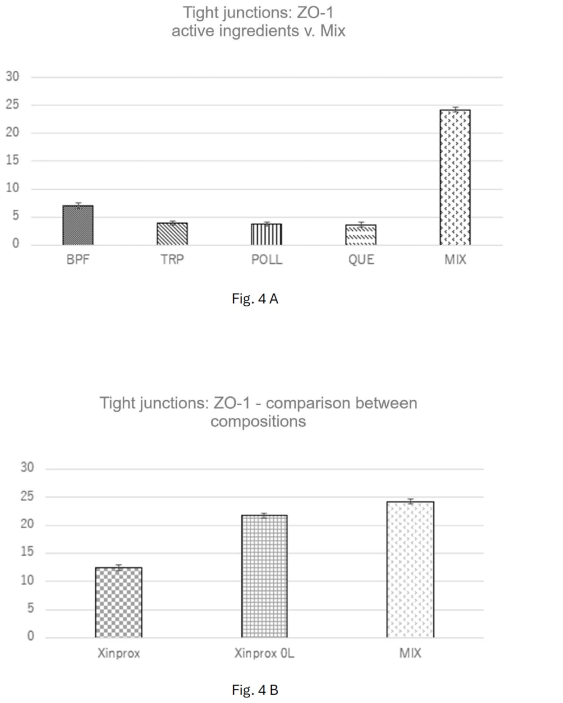

Figure 4 shows the integrity of the tight junctions, in particular of Zonula occludens, in the mixture compared to A) the individual active ingredients and B) compared to the comparison compositions indicated in the experimental part. The mix is demonstrated to be statistically superior p<0.05 compared to the other two mixtures.

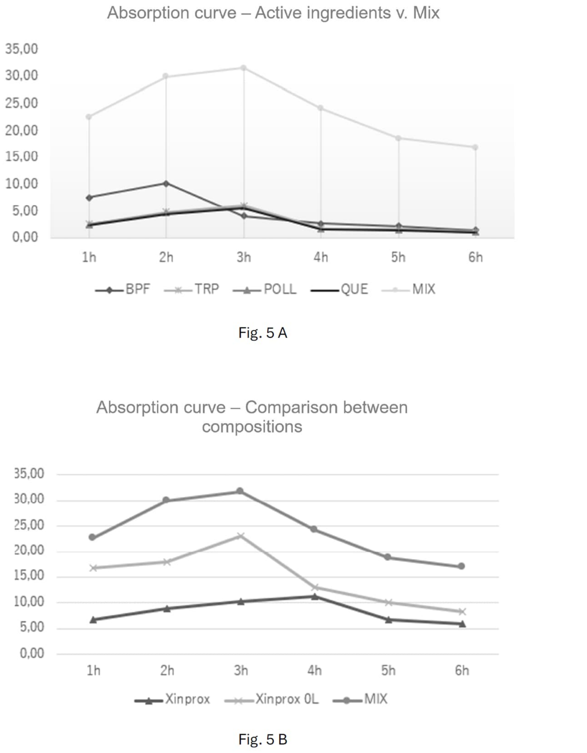

Figure 5 shows absorption through the intestinal barrier compared to A) the individual active ingredients and B) compared to the comparison compositions indicated in the experimental part. The mix is demonstrated to be statistically superior p<0.05 compared to the other two mixtures.

Figures 6 to 10 show the results of Phase 2 of the experiment described in the experimental part.

Figure 6 shows the viability of the mixture compared to A) the individual active ingredients and B) compared to the comparison compositions indicated in the experimental part. The mix is demonstrated to be statistically superior p<0.05 compared to the other two mixtures.

Figure 7 shows the expression of p75 of the mixture compared to A) the individual active ingredients and B) compared to the comparison compositions indicated in the experimental part. The mix is demonstrated to be statistically superior p<0.05 compared to the other two mixtures.

Figure 8 shows the levels of MPZ of the mixture compared to A) the individual active ingredients and B) compared to the comparison compositions indicated in the experimental part. The mix is demonstrated to be statistically superior p<0.05 compared to the other two mixtures.

Figure 9 shows the levels of NRG1 of the mixture compared to A) the individual active ingredients and B) compared to the comparison compositions indicated in the experimental part. The mix is demonstrated to be statistically superior p<0.05 compared to the other two mixtures.

Figure 10 shows the levels of ERB3 of the mixture compared to A) the individual active ingredients and B) compared to the comparison compositions indicated in the experimental part. The mix is demonstrated to be statistically superior p<0.05 compared to the other two mixtures.

### Detailed description of the invention

An object of the present invention is a mixture that comprises or, alternatively, consists of: (a) a *Secale cereale* pollen extract (L.; or rye) (i.e. preferably, Graminex^{®} G63^{®}), (b) a quercetin, (c) a bergamot extract, and (d) a tryptophan. Preferably, said mixture of the invention can comprise or, alternatively, consist of: (a) a *Secale cereale* pollen extract (L.; or rye) (i.e. preferably, Graminex^{®} G63^{®}), (b) a quercetin, (c) a bergamot extract, and (d) tryptophan and/or a Saffron extract.

Preferably, the mixtures and compositions that comprise said mixtures do not contain or include lipoic acid or its derivatives or salts.

Preferably, lipoic acid is understood to include a lipoic acid, both in oxidated and reduced form, and/or a pharmaceutically acceptable salt thereof and/or their mixtures.

The mixture of the invention comprises (a) a *Secale cereale* pollen extract (L.; or rye).

Preferably, said (a) rye *(Secale cereale* L.) pollen extract can comprise a water-soluble pollen extract (hydrophilic fraction) in an amount by weight comprised from 10% to 40%, preferably comprised from 20% to 30%, for example 25%, a fat-soluble pollen extract (lipophilic fraction) in an amount by weight comprised from 0.1% to 5%, preferably comprised from 1% to 3%, for example 1.5%, and excipients to create a flowing powder that are chosen, for example, from one or more of the following: maltodextrin, microcrystalline cellulose, silicon dioxide, calcium stearate, calcium phosphate and gum arabic, in an amount by weight comprised from 55% to 89.9% with respect to the total weight of the pollen extract.

Preferably, said (a) rye *(Secale cereale* L.) pollen extract can preferably be a rye *(Secale cereale* L.) pollen extract which can further contain a maize *(Zea mays* L.) extract and/or a timothy *(Phleum pratense* L.) extract. Preferably, said (a) rye *(Secale cereale* L.) pollen extract (or dry extract) and, optionally, maize *(Zea mays* L.) extract and/or a timothy *(Phleum pratense* L.) extract can, without constraints or limitations, be, for example, a product present on the market of the Graminex^{®} G63^{®} type (or Flower Pollen ExtractTM, manufactured by Graminex^{®} L.L.C., USA) or similar, which may contain by weight, for example, around 25% a water-soluble pollen extract, around 1.5% a fat-soluble pollen extract and around 73.5% excipients to create a powder.

Said commercial product called Graminex^{®} G63^{®} can comprise a standardised rye *(Secale cereale* L.) pollen extract and, optionally, a maize *(Zea mays* L.) extract and/or a timothy *(Phleum pratense* L.) extract (briefly, pollen extract), for example, in the form of a powder comprising a water-soluble fraction (soluble in water) and a fat-soluble fraction (not soluble in water) in a variable ratio by weight, for example, comprised from 40:1 a 5:1, preferably comprised from 30:1 to 10:1, more preferably comprised from 20:1 to 15:1, for example it may contain a water-soluble fraction:fat-soluble fraction equal to around 20:1 by weight, wherein the water-soluble fraction is standardised in amino acids and the fat-soluble fraction is standardised in phytosterols.

In the context of the present invention, "phytosterols" is intended to mean a group of sterols present in *Secale cereale* and, optionally, *Zea mays* and *Phleum pratense* plants. The main phytosterols are: cholesterol, β-sitosterol, campesterol, stigmasterol, brassicasterol, delta 5-avenasterol, colestanol, sitostanol, campestanol, erythrodiol, uvaol, betulin, cholesterol, 24-methylene cholesterol, delta-5-24-stigmastadienol, delta-7-stigmastenol, delta-7-avenasterol, delta-7-campesterol, delta-5-23-stigmastadienol. For example, the commercial product Graminex^{®} can have the following characteristics: preferably a water-soluble fraction:fat-soluble fraction in a ratio by weight of 20:1, preferably a bulk density comprised from 0.30 to 0,70 g/cc (reference USP 616), preferably a pH value comprised from 3.5 to 5.8 (reference USP 791), preferably a Loss on dry: not higher than 6.0% (reference GM 078); preferably a particle dimension: not lower than 95% through 80 mesh (reference GM 075); it may further comprise the following additives/excipients: maltodextrin, microcrystalline cellulose, silicon dioxide, calcium stearate, monocalcium phosphate and gum arabic.

For example, said (a) pollen extract (i.e. preferably, Graminex^{®} G63^{®}) present in the mixture of the present invention (together with (b), (c) and (d)) has an alpha-amino acids content not lower than around 3.6 mg/250 mg (preferably from 3.6 mg to 200 mg, for example 5 mg, 10 mg, 20 mg, 30 mg, 40 mg, 50 mg, 60 mg, 70 mg, 80 mg, 90 mg, 100 mg, 120 mg, 140 mg, 160 mg, 180 mg, on 250 mg) and a phytosterol content not lower than around 0.2 mg/250 mg (preferably from 0.2 mg to 10 mg, for example 0.5 mg, 1 mg, 2 mg, 3 mg, 4 mg, 5 mg, 6 mg, 7 mg, 8 mg or 9 mg, on 250 mg).

The amino acid and/or phytosterol content present in said pollen extract can, for example, be determined by means of titration methods (for example, titration using the UV or HPLC technique) and standard equipment known to a person skilled in the art. Said Graminex^{®} (i.e. *Secale cereale* pollen extract and, optionally, *Zea mays* and/or *Phleum pratense* extract) is obtained, for example, by extracting from the rye (and optionally, maize and/or timothy) separately the soluble part (G60) and the lipophilic part (GFX), to give the product G63^{®} that comprises a combination G60:GFX=20:1, as the ratio by weight.

In the context of the present invention, "dry extract" is intended as an extract in powder form having a water content in percentage by weight from 0.05% to 15% (for example, 0.1%, 0.5%, 1%, 2%, 3%, 4%, 5%, 6%, 7%, 8%, 9%, 10%, 11%, 12%, 13%, or 14%), preferably from 0.05% to 10% or from 0.05% to 5%.

In the context of the present invention, the quantities expressed by weight or percentage volume have their own natural tolerance, even if not expressly indicated, given by the method, equipment used or by the experience of the operator who has performed the measurements, and the tolerance to be considered can, for example, be in the order of ±0.05% up to ±0.1%, even if not expressly indicated.

*Secale cereale* (L., 1753), also known as *Rye,* is a cereal widely present in temperate climates; scientific classification: *Plantae* kingdom, *Tracheobionta* subkingdom, *Spermatophyta* superdivision, *Magnoliophyta* division, *Liliopsida* class, *Cyperales* order, *Poaceae* family, rye genus.

*Zea mays* (L., 1753), also known as maize, is an annual herbaceous plant of the Poaceae family; scientific classification: *Plantae* kingdom, *Magnoliophyta* division, *Liliopsida* class, *Poales* order, *Poaceae* family, *Panicoideae* subfamily, *Andropogoneae* tribe, Zea genus.

*Phleum pratense* (L., 1753), also known as timothy, meadow cat's-tail or timothy-grass, is a plant of the *Poaceae* family; scientific classification: *Plantae* kingdom, *Tracheobionta* subkingdom, *Magnoliophyta* division, *Liliopsida* class, *Cyperales* order, *Poaceae* family, *Pooideae* subfamily, *Aveneae* tribe, *Phleum* genus.

Preferably, said mixture of the invention comprises or, alternatively, consists of: (a) a *Secale cereale* pollen extract, and, optionally, a *Zea mays* and/or *Phleum pratense* extract comprising a water-soluble fraction and a fat-soluble fraction in a ratio by weight comprised from 40:1 to 5:1, preferably comprised from 30:1 to 10:1, more preferably comprised from 20:1 to 15:1, wherein the water-soluble fraction is standardised in amino acids and the fat-soluble fraction is standardised in phytosterols (i.e. preferably, Graminex^{®} G63^{®}), (b) quercetin, (c) bergamot extract, and (d) tryptophan.

The mixture of the invention comprises, in addition to (a) a *Secale cereale* pollen extract (L.; or rye), also (b) a quercetin.

In the context of the present invention, the term "quercetin" is intended to mean both quercetin as such (with various degrees of purity, for example from 70% to 99.9% weight/weight) or, alternatively, a plant extract (botanical) comprising (or titrated, using methods and equipment known to a person skilled in the art) quercetin (for example, titrated from 50% to 99.9% weight/weight). Quercetin (IUPAC name 3,3',4',5,7-pentahydroxyflavone, example CAS no. 6151-25-3) is a flavonoid. An example of quercetin usable in the mixture and composition of the present invention is quercetin in the form of a *Sophorae japonica* extract, obtained using methods and equipment known to a person skilled in the art.

An example of quercetin usable in the mixture and composition of the present invention is the commercial product "Quercetin dihydrate powder" sold by Shangai Freemen. Said product is a yellow powder with a particle size passing through an 80 mesh, bulk density ≥ 0.20 g/ml and lower than 2, tapped density ≥ 0.20 g/ml and lower than 2, preferably ≥ 0.30 g/ml and lower than 2. Said product comprises quercetin having a degree of purity from 70% to 99.9% weight/weight, preferably ≥ 90% weight/weight, more preferably ≥ 95% weight/weight.

The mixture of the invention comprises, in addition to (a) a *Secale cereale* pollen extract (L.; or rye), (b) a quercetin, also (c) a bergamot extract.

Preferably, the bergamot extract in the mixture of the invention is a bergamot extract of the *Citrus Bergamia Risso et Poit* extract type.

In the context of the present invention, "bergamot extract" is intended as including all types of *Citrus Bergamia Risso et Poit.* extracts comprising at least one compound chosen from the group comprising or, alternatively, consisting of naringin, (neo)hesperidin and neoeriocitrin, regardless of the concentration or extraction method thereof.

An example of a commercial product of *Citrus aurantium L. var. bergamia* extract is Bergavit^{™} sold by Bionap, consisting of a fruit extract obtained from *Citrus aurantium Bergamia* and having CAS number 89957-91-5 and maltodextrins (CAS number 9050-36-6). Such bergamot extract has a flavonoid content (neoeriocitrin, naringin, neohesperidin) comprised from 25% to 28% w/w measured by HPLC.

Preferably, the bergamot extract in the mixture of the invention comprises an amount of total flavonoids comprised from 10% to 60%, preferably comprised from 20% to 50%, more preferably from 30% to 40% by weight, relative to the total weight of the extract, wherein said flavonoids comprise at least a compound chosen from the group comprising or, alternatively, consisting of naringin, neohesperidin, neoeriocitrin, melitidin, and brutieridin.

Preferably, the bergamot extract in the mixture of the invention comprises or, alternatively, consists of naringin, neohesperidin, neoeriocitrin, melitidin and brutieridin.

Preferably, the bergamot extract in the mixture of the invention comprises or, alternatively, consists of naringin, neohesperidin and neoeriocitrin.

In the context of the present invention, the bergamot extract has a total flavonoid content comprised from 10% to 60%, preferably comprised from 20% to 50%, more preferably comprised from 30% to 40%, for example 31%, 32%, 33%, 34%, 35%, 36%, 37%, 38%, 39% by weight, with respect to the total weight of the extract which contains, among other things, also flavonoids that preferably comprise at least one compound chosen from the group comprising or, alternatively, consisting of naringin, neohesperidin, neoeriocitrin, melitidin and brutieridin.

Preferably, said bergamot extract in the mixture of the invention may comprise the active compound naringin. Preferably, naringin can be present in said bergamot extract in an amount by weight comprised from 6% to 14%, preferably comprised from 8% to 13%, more preferably comprised from 10% to 12%, for example 11% by weight, relative to the total weight of the bergamot extract.

Preferably, in addition to naringin, said bergamot extract in the mixture of the invention can further comprise the active compound neohesperidin. Preferably, neohesperidin can be present in said bergamot extract in an amount by weight comprised from 6% to 14%, preferably comprised from 8% to 13%, more preferably comprised from 11% to 12%, relative to the total weight of the bergamot extract.

Preferably, said bergamot extract in the mixture of the invention can comprise the naringin compound in an amount by weight comprised from 6% to 14%, preferably comprised from 8% to 13%, more preferably comprised from 10% to 12%, for example 11% by weight, relative to the total weight of the bergamot extract, and the neohesperidin compound in an amount by weight comprised from 6% to 14%, preferably comprised from 8% to 13%, more preferably comprised from 11% to 12%, relative to the total weight of the bergamot extract.

Preferably, said bergamot extract in the mixture of the invention can also comprise, in addition to naringin and neohesperidin, also the neoeriocitrin compound, preferably in the percentage amounts specified above. Preferably, neoeriocitrin is present in the bergamot extract in an amount by weight comprised from 6% to 14%, preferably comprised from 8% to 12%, more preferably comprised from 10% to 11% by weight, relative to the total weight of the bergamot extract.

Preferably, said bergamot extract in the mixture of the invention can also comprise, in addition to naringin, neohesperidin and neoeriocitrin, also the melitidin compound, preferably in the percentage amounts specified above. Preferably, melitidin is present in the bergamot extract in the mixture of the invention in an amount by weight comprised from 0.5% to 3%, more preferably comprised from 1% to 2%, for example 2% by weight, relative to the total weight of the bergamot extract.

Preferably, said bergamot extract in the mixture of the invention can also comprise, in addition to naringin, neohesperidin, neoeriocitrin and melitidin, the brutieridin compound, preferably in the percentage amounts specified above. Preferably, brutieridin is present in the bergamot extract in the mixture of the invention in an amount by weight comprised from 2% to 8%, preferably comprised from 3% to 6%, more preferably comprised from 4% to 5% by weight, relative to the total weight of the bergamot extract.

For example, a bergamot extract can be used that is preferably obtained from *Citrus Bergamia Risso et Poit.,* for example in powder form, which, in water, can have a pH value comprised from 3 to 4, for example a value of 3.5, with an *Average Mesh Size* of around 60 mesh, a *Bulk Density* comprised from 40 to 60 g/100 ml, a humidity content lower than 12%, for example a humidity value comprised from lower than 8% to higher than 2%, for example 3.4%, and a good water solubility at 40°C and in 50% water+ethanol.

For example, a bergamot extract can be used that is preferably obtained from *Citrus Bergamia Risso et Poit.,* for example in powder form, having a total content of flavonoids chosen from naringin, neohesperidin, neoeriocitrin, melitidin and brutieridin comprised from 10% to 60%, preferably comprised from 20% to 50%, more preferably comprised from 30% to 40% by weight, relative to the total weight of the bergamot extract.

For example, a bergamot extract can be used that is preferably obtained from *Citrus Bergamia Risso et Poit.,* for example in powder form, having a total content of flavonoids chosen from naringin, neohesperidin, neoeriocitrin, melitidin and brutieridin of around 40±2% by weight in total flavonoids, in which, for example, said at least one flavonoid present in said c) bergamot extract may, preferably, be contained in an amount by weight comprised:
- from 5% to 40% of naringin, preferably comprised from 20% to 30%, more preferably comprised from 28% to 29% by weight, with respect to the total weight of the flavonoids;
- from 5% to 40% of neohesperidin, preferably comprised from 20% to 35%, more preferably comprised from 25% to 30% by weight, with respect to the total weight of the flavonoids;
- from 5% to 40% of neoeriocitrin, preferably comprised from 20% to 30%, more preferably comprised from 25% to 26% by weight, with respect to the total weight of the flavonoids;
- from 1% to 10% of melitidin, preferably comprised from 3% to 7%, more preferably comprised from 4% to 5% by weight, with respect to the total weight of the flavonoids;
- from 5% to 20% of brutieridin, preferably comprised from 10% to 15%, more preferably comprised from 11% to 12% by weight, with respect to the total weight of the flavonoids.

More preferably, the bergamot extract present in Mix 1 comprises in an amount by weight, with respect to the weight of total flavonoids:
- from 28% to 29% naringin, e.g. 28.7%; and/or
- from 29% to 30% (neo)hesperidin, e.g. 29.2%; and/or
- from 24% to 26% neoeriocitrin, e.g. 25.7%; and/or
- from 3% to 6% melitidin, e.g. 4.7%; and/or
- from 10% to 15% brutieridin, e.g. 11.3%.

An example of a commercial product of a *Citrus Bergamia Risso et Poit* extract is BERGAMOT POLYPHENOLIC FRACTIONTM (BPFTM), for example sold by H&AD S.r.l., comprising a total flavonoid content measured by HPLC comprised from 20% to 60%, preferably comprised from 20% to 50%, more preferably from 30% to 40%, for example 39%, of which: from 6% to 14%, preferably from 8% to 12%, more preferably 10.2% neoeriocitrin; from 6% to 14%, preferably from 8% to 13%, more preferably 11% naringin; from 6% to 14%, preferably from 8% to 13%, more preferably from 11% to 12% neohesperidin; from 0.5% to 3%, preferably from 1% to 2%, more preferably 2% melitidin and from 2% to 8%, preferably from 3% to 6%, more preferably from 4% to 5% brutieridin, by weight with respect to the total weight of the bergamot extract.

In other words, if the total amount of flavonoids in the bergamot extract is, for example, equal to around 40%, this can, for example, be divided as follows: 10% is the amount of neoeriocitrin, 12% is the amount of naringin, 12% is the amount of neohesperidin, 2% is the amount of melitidin, and 4% is the amount of brutieridin.

More preferably, the bergamot extract in Mix 1 comprises, by weight, relative to the weight of total flavonoids:
from 6% to 40%, preferably from 15% to 35%, more preferably from 20% to 30%, for example 28% or 29%, naringin, from 6% to 40%, preferably from 15% to 35%, more preferably from 20% to 30%, for example 29% or 30% neohesperidin, from 6% to 40%, preferably from 15% to 35%, more preferably from 20% to 30%, for example 26% neoeriocitrin, from 0.5% to 10%, preferably from 1% to 8%, more preferably from 3% to 6%, for example 5% melitidin, and from 0.5% to 25%, preferably from 5% to 20%, more preferably from 10 to 15%, for example 11% brutieridin.

The bergamot extract appears as a yellow-coloured powder having particle size passing through a 60 mesh sieve and a bulk density comprised, for example, from 30 to 70 g/100 ml, preferably comprised from 40 to 60 g/100 ml, more preferably 55 g/100 ml, 56 g/100 ml, 57 g/100 ml, 58 g/100 ml, 59 g/100 ml (DIN/ISO 697).

Preferably, the BERGAMOT POLYPHENOLIC FRACTIONTM (BPFTM), for example sold by H&AD S.r.l. comprises a total flavonoid content measured by HPLC of 39.7%, of which 10.2% neoeriocitrin, 11.4% naringin, 11.6% neohesperidin, 1.9% melitidin and 4.5% brutieridin. Said product contains a bergamot extract obtained from fruit juice, but could also be obtained from the rind, from the tree and from the zest. It is in the form of a yellow powder with typically citrus odour and flavour, with particle size passing through a 60 mesh.

Another example of a commercial product is BergavitTM sold by Bionap, consisting of a fruit extract obtained from *Citrus aurantium Bergamia* and having CAS number 89957-91-5. Such bergamot extract has a flavonoid content (neoeriocitrin, naringin, neohesperidin) from 25% to 28% w/w measured by HPLC.

A further example of a commercial product is KALITA sold by Giellepi, formed of a phytocomplex extracted from the *Citrus bergamia var. Risso* whole fruit, containing a total amount of polysaccharides comprised from 10% to 60%, preferably from 20% to 50%, more preferably from 25% to 40% by weight, and a total amount of flavonoids comprised from 10% to 30%, more preferably from 15% to 25% by weight.

The mixture of the invention comprises (a) a *Secale cereale* pollen extract (L.; or rye), (b) a quercetin, (c) a bergamot extract, and d) tryptophan and/or a Saffron extract.

The tryptophan can, for example, by the type having IUPAC name L-tryptophan, such as, for example, having a CAS N.° 73-22-3.

The component d), in the mixture a) + b) + c), can comprise a tryptophan, in the form L-tryptophan and/or a saffron extract. If the component d) comprises both the tryptophan and the saffron extract, these are present in a weight ratio comprised from 1:5 to 5:1, preferably comprised from 1:3 to 3:1, for example 1:1.

Preferably, the saffron extract can be a *Crocus sativus* (L.) extract, commonly called saffron, which is a perennial plant in the *Iridaceas* family. The stigma (upper part of the pistil on which the pollen grain rests) of *Crocus sativus* contains numerous bioactive compounds, including alpha-crocetin, crocin, picrocrocin and safranal. Extraction can, for example, be performed through extraction in water or water/ethanol (30%-50% ethanol) with the methods, techniques and equipment known in the art.

In the context of the present invention, d) an extract of *Crocus sativus* is understood to include all types of *Crocus sativus* stigma extracts, comprising at least crocin and/or safranal. Preferably, the *Crocus sativus* extract is a dry extract titrated in crocin and/or safranal, the minimum safranal content being ≥ 0.30% w/w and/or crocin content being ≥ 0.40% w/w, the safranal content preferably being in a range from 0.34% to 0.45% by weight relative to the total weight of the dry extract and/or the crocin content in a range from 0.45% to 0.60% by weight relative to the total weight of the dry extract.

A type of saffron extract that can be present in the mixture according to the invention can be, for example, the commercial product SATIEREAL^{®} sold by Natac, which is a dry extract of saffron stigma *(Crocus sativus* L.) characterised by a minimum saffron content of ≥ 0.1% lower than 5%, preferably ≥ 0.2% lower than 3%, for example comprised from 0.3% a 2% weight/weight, measured by spectrophotometry, and/or a minimum crocin content of ≥ 0.1% lower than 5%, preferably ≥ 0.2% lower than 3%, for example 0.4% - 2% w/w measured by HPLC, and/or a minimum picrocrocin content of ≥ 0.1% lower than 5%, preferably ≥ 0.2% lower than 3%, for example comprised from 0.3% to 2% w/w measured by HPLC. Another commercial product is Affron^{®} sold by Pharmactive Biotech Products. Said product is a dry extract of saffron stigma *(Crocus sativus L.)* which is in the form of an orange powder with particle size of around 60 mesh, a bulk density > 0.3 g/mL, for example > 0.3 g/mL lower than 2 g/mL.

An object of the present invention is a composition comprising the mixture of the present invention comprising or, alternatively, consisting of (a), (b), (c) and (d) (as defined in the present description) and at least one acceptable pharmaceutical or food grade additive and/or excipient.

The mixtures or compositions of the invention are advantageously formulated for oral (or sublingual) administration.

The dosage form of the mixture or composition of the invention can be a solid form, such as a tablet, chewable tablet, effervescent tablet, capsule, soft capsule, lozenge, granules or powder (granules or powder to be dissolved in water or orodispersible), or a semi-solid form, such as soft gel, or a liquid form, such as a solution, suspension, dispersion, emulsion or syrup; preferably, the composition of the invention is in solid form for oral use, more preferably in tablet form.

The mixture or composition of the invention can advantageously comprise for the "daily dose unit", preferably in solid form (e.g. one or more tablets, capsules or granules/powder dosed in sachets), the following quantities:
(a) a *Secale cereale* pollen extract, preferably comprising a hydrophilic fraction:lipophilic fraction, in a ratio by weight comprised from 40:1 to 5:1, preferably comprised from 30:1 to 10:1, more preferably comprised from 20:1 to 15:1, e.g. equal to about 20:1 by weight, preferably in an amount by weight comprised from 50 mg to 2000 mg (for example, 100 mg, 200 mg, 300 mg, 400 mg, 500 mg, 600 mg, 700 mg, 800 mg, 1000 mg, 1200 mg, 1400 mg, 1600 mg, or 1800 mg), more preferably comprised from 500 mg to 1000 mg (for example, 1000 mg),
(b) a quercetin in an amount by weight comprised from 10 mg to 400 mg (for example, 20 mg, 40 mg, 60 mg, 80 mg, 100 mg, 120 mg, 140 mg, 160 mg, 180 mg, 200 mg, 250 mg, 300 mg, or 350 mg), preferably comprised from 100 mg to 200 mg (for example, 200 mg), considering that 200 mg/day is the maximum daily dose for an adult currently permitted by Italian regulations;
(c) a bergamot extract in an amount by weight comprised from 10 to 1,000 mg, preferably comprised from 200 to 600 mg, more preferably from 300 to 400 mg of bergamot extract (wherein said bergamot extract preferably has a total flavonoid content (neoeriocitrin, naringin, neohesperidin, melitidin, brutieridin) comprised from 10% to 60%, preferably from 20% to 50%, more preferably from 30% to 40% w/w);
(d) a tryptophan, preferably in the L-tryptophan form, in an amount comprised from 10 mg to 600 mg (for example, 50 mg, 100 mg, 150 mg, 180 mg, 200 mg, 220 mg, 250 mg, 280 mg, 300 mg, 400 mg, 450 mg, 500 mg, or 550 mg), preferably from 150 mg to 300 mg (for example, around 300 mg).

Preferably, said mixture or composition of the invention can advantageously comprise for the "daily dose unit", preferably in solid form (e.g. one or more tablets, capsules or granules/powder dosed in sachets), the following quantities:
(a) a Secale cereale pollen extract, preferably comprising a hydrophilic fraction:lipophilic fraction, in a ratio by weight comprised from 40:1 to 5:1, preferably comprised from 30:1 to 10:1, more preferably comprised from 20:1 to 15:1, e.g. equal to about 20:1 by weight, in an amount comprised from 500 mg to 1000 mg;
(b) a quercetin in an amount by weight comprised from 100 mg to 200 mg;
(c) a bergamot extract in an amount by weight comprised from 300 mg to 600 mg; preferably, said bergamot extract has a total flavonoid content (neoeriocitrin, naringin, neohesperidin, melitidin, brutieridin) comprised from 10% to 60%, preferably comprised from 20% to 50%, more preferably comprised from 30% to 40% by weight);
(d) a tryptophan, preferably in the form of L-tryptophan, in an amount by weight comprised from 150 mg to 300 mg and, optionally, additives and excipients.

The dosages indicated above are to be understood as a dosage unit and not as the daily amount. The daily amount, or daily dose unit, can be administered by means of one or more dosage units.

Said "daily dose unit" of the composition of the invention can be administered to an individual in need within a 24-hour interval with a single dose or divided into 2, 3 or 4 doses spaced apart at time intervals of 4 hours to 12 hours, depending on the type of dosage form and the individual's needs. Preferably, said "daily dose unit" of the composition of the invention is administered to an individual in need once a day (for example, between meals) in the form of a tablet.

The mixture of the invention can advantageously comprise for the "daily dose unit" or "administration dose unit", preferably in solid form (e.g. one or more tablets, capsules or granules/powder dosed in sachets), the following percentages by weight:
(a) a *Secale cereale* pollen extract, preferably comprising a hydrophilic fraction:lipophilic fraction, in a ratio by weight comprised from 40:1 to 5:1, preferably comprised from 30:1 to 10:1, more preferably comprised from 20:1 to 15:1, e.g. equal to about 20:1 by weight, in an amount comprised from 20% to 80% (for example, 30%, 35%, 40%, 45%, 50%, 55%, 60%, 65%, 70%, or 75%), preferably from 40% to 60% (for example, around 45-50%),
(b) quercetin in an amount comprised from 1% to 30% (for example, 2%, 5%, 10%, 15%, 20%, or 25%), preferably from 5% to 15% (for example, around 9-10%);
(c) a bergamot extract in an amount by weight comprised from 5% to 60% (for example, 10%, 15%, 20%, 25%, 30%, 35%, 40%, 45%, 50%, or 55%), preferably from 10% to 40% (for example, around 28-30%);
(d) a tryptophan, preferably in the form of L-tryptophan, in an amount by weight comprised from 1% to 50% (for example, 10%, 15%, 20%, 25%, 30%, 35%, 40%, or 45%), preferably from 5% to 30% (for example, around 14-15%).

An object of the present invention is the mixture or composition of the invention (comprising (a), (b), (c), and (d), according to any of the embodiments or aspects described) for use as a medicament.

An object of the present invention is the mixture or composition of the invention (comprising (a), (b), (c), and (d), according to any of the embodiments or aspects described) for use in a method of treatment, preventive and/or curative, of prostate pathologies and/or symptoms, preferably wherein said prostate pathologies and symptoms are selected from non-bacterial prostatitis/chronic pelvic pain syndrome (CP/CPPS), benign prostate hyperplasia (BPH), and symptoms and/or disorders connected to said non-bacterial prostatitis/chronic pelvic pain syndrome (CP/CPPS) and benign prostate hyperplasia (BPH) in an individual having need, through the administration of a therapeutically effective amount of the mixture or composition of the present invention.

Symptoms and/or disorders connected to said non-bacterial prostatitis/chronic pelvic pain syndrome (CP/CPPS) and benign prostate hyperplasia (BPH) are intended to mean, for example: constant pain in the scrotum and anus or central-lower abdomen; pain during or after urination; pain during or after ejaculation; frequent urination, both during the day (pollakiuria) and during the night (nocturia); sensation of incomplete emptying of the bladder after urination; urgency and impossibility of delaying urination; erectile dysfunction and decreased sexual desire, and others known to a person skilled in the art. The mixture or composition of the invention can be for use in a treatment method, preventive or curative, of prostate pathologies and/or symptoms (as defined above), both when administered to an individual as a single therapy and when administered as an adjuvant of at least one other therapy or composition capable of treating said pathologies and/or symptoms.

For clarity, in order to achieve the object of the present invention, the active compounds of the mixture or composition of the present invention ((a), (b), (c), and (d)) can also be administered separately or in groups (preferably in a time interval from 30 minutes to 2-3 hours) and in any order.

The mixture or composition of the invention can be for use in a treatment method, preventive or curative, of prostate pathologies and/or symptoms (as defined above), both when administered to an individual as a single therapy and when administered as an adjuvant of at least one other therapy or composition capable of treating said pathologies and/or symptoms.

An object of the present invention is a treatment method, preventive and/or curative, of prostate pathologies and/or symptoms, as described, in an individual having need, through the administration to said individual of a therapeutically effective amount of the mixture or composition of the present invention.

An object of the present invention is the use for a non-medical (non-therapeutic) purpose, for conditions or sensations in an individual connected to prostate problems, through the administration of an adequate amount of the mixture or composition of the present invention to said individual.

For clarity, in order to achieve the object of the present invention, the active compounds of the mixture or composition of the present invention ((a), (b), (c), and (d)) can also be administered separately or in groups (preferably in a time interval from 30 minutes to 2-3 hours) and in any order.

Said at least one pharmaceutical or food grade additive and/or excipient, comprised in the composition of the invention together with the mixture of (a), (b), (c), and (d), consists of a substance devoid of therapeutic activity, suitable for pharmaceutical or food use, and selected from the auxiliary substances known to the person skilled in the art, e.g. diluents, solvents (including water, glycerine and ethyl alcohol), solubilisers, thickeners, sweeteners, flavourings, colourings, lubricants, surfactants, antimicrobials, antioxidants, preservatives, pH stabilising buffers and mixtures thereof. Non-limiting examples of such substances are phosphate buffers (for example, dicalcium phosphate), a stearate of an alkali or alkaline earth metal (for example magnesium), silicon dioxide, mono- and diglycerides of fatty acids, microcrystalline cellulose, hydroxypropyl cellulose, hydroxypropyl methylcellulose, starch or corn starch, and natural or artificial flavourings (for example, iron oxides).

Said composition of the invention can be a pharmaceutical composition, a composition for medical devices (Medical Device Regulation (EU) 2017/745 (MDR)), a dietary supplement and/or a food for special medical purposes (FSMP).

In the context of the present invention, the term "individual(s)" indicates mammals (animals and humans), preferably human beings.

The term "therapeutically effective amount" refers to the amount of mixture or compound or formulation that elicits the biological or medicinal response in a tissue, system or individual that is sought and defined by the person skilled in the art.

Unless otherwise specified, the expression "mixture or composition" that comprises a component in an amount "comprised in a range from x to y" means that said component can be present in the mixture or composition in all the amounts present and comprised within in said range, even if not explicitly stated, including the endpoints of the range.

### Examples

### Mixture 1 with ingredient mg/dose:

*(a) Secale cereale* pollen extract (example, with a hydrophilic fraction:lipophilic fraction = 20:1) 500 mg;
(b) quercetin 100 mg;
(c) bergamot extract 300 mg;
(d) tryptophan 150 mg; and, optionally,
additives/excipients in a sufficient amount.

### Mixture 2 with ingredient mg/dose:

*(a) Secale cereale* pollen extract (example, with a hydrophilic fraction:lipophilic fraction = 20:1) 750 mg;
(b) quercetin 150 mg;
(c) bergamot extract 450 mg;
(d) tryptophan 200 mg; and, optionally,
additives/excipients in a sufficient amount.

### Mixture 3 with ingredient mg/dose:

*(a) Secale cereale* pollen extract (hydrophilic:lipophilic = 20:1) 1000g;
(b) quercetin 200 mg;
(c) bergamot extract 600 mg;
(d) tryptophan 300 mg; and, optionally,
additives/excipients in a sufficient amount.

### Experimental part

Neuro-inflammation is a key component of the pathogenesis of prostate diseases. In fact, inflammation of the prostate gland, present in the same way in both prostatitis and in benign prostatic hyperplasia, also extends to the level of the neural tissue that surrounds the prostate, causing one of the most difficult symptoms to treat, which is neuropathic pain in the pelvic area.

The applicant has performed the experiments described here below, in order to demonstrate that the mixture according to the invention (hereinafter, indicated as "MIX") effectively counters neuro-inflammation, and to a greater extent than the individual active ingredients and the mixture comprising lipoic acid in place of a bergamot extract.

### Materials and methods

### Experimental groups tested

MIX: BPF + TRP + POLL + QUE
XINPROX: TRP + POLL + QUE + ALA (Lipoic Acid)
XINPROX 0L: TRP + POLL + QUE
Individual active ingredients: Bergamot (BPF) - L-tryptophan (TRP) - Rye pollen (POLL) - Quercetin (QUE).
GGF: negative, simulates pathological state.

The analysis was performed at 1, 2, 3, 4, 5, 6 h and 24h.

### Step 1. Analysis of absorption and bioavailability through the intestinal barrier

For performance of the absorption and bioavailability study, it is planned to use a 3D barrier model validated in literature, as the best experimental strategy. To perform the study, it is necessary to use well-designed *in vitro* models, called Transwell^{®}, which provide for the use of human intestinal epithelial cells on absorption of compounds taken orally, having 90% compatibility with humans doi:10.3390/biomedicines9111543. Therefore, the experimental model provides for use of the CaCo2 cell line, broadly used and validated, as the human intestinal epithelial cells model for absorption studies on compounds taken orally.

The cells seeded on the Transwell^{®} insert were maintained in a complete medium changed every other day for 21 days before the stimulation, in order to ensure the maturation and formation of intestinal microvilli. Maturation was complete when a transepithelial resistance (TEER) value ≥400Ω·cm2 was reached.

When cultured on Transwell^{®} supports for 21 days, the cells polarise and differentiate to form a continuous epithelium (monolayer) with functional tight junctions and brush border microvilli. The cell monolayer provides a physical and biochemical barrier to the passage of ions and small molecules, separating the model into two separate compartments: one apical, corresponding with the human intestinal lumen, and one basolateral, which corresponds with the blood circulation compartment of the organism (pH7.4). This model will be used to assess the degree of permeability, excluding irritability or damage to the intestinal epithelium, both before and after taking the substances in question, analysing possible ROS production and the inflammation pathways. In particular, permeability will be analysed by measuring the transepithelial/transendothelial electrical resistance (TEER), a widely accepted quantitative technique that uses a volt/ohm meter (EVOM3) to measure the integrity of the dynamics of tight junctions, by evaluating the integrity of the cellular barriers following the passage of the substances being tested. Furthermore, the changes in the expression of tight junction (TJ) proteins are crucial and typically evaluated in order to analyse the barrier function, since, behaving like multiple protein complexes, they form a selectively permeable seal between adjacent epithelial cells and, for this reason, they will be analysed as indicators of the integrity of the epithelium, in particular by analysing occludin, claudin and zonula occludens.

### Step 2. Analysis of the main mechanisms involved in the neuropathies

Starting from intestinal passage, a further *in vitro* 3D model will be created for study of the peripheral nerve. Such model, called 3D *"engineered neural tissue"* (EngNT), allows researchers to bridge the gap between the models obtained in the *in vitro* monolayer models and the studies on humans more successfully, to the extent that preclinical studies with 3D models are the basis for determining the efficacy and safety of the compounds required by regulatory agencies, including, for example, the *Medicines and Healthcare Products Regulatory Agency* (MHRA) or the *US Food and Drug Administration* (FDA). It should be considered that, in order to obtain successful regeneration following nerve lesions, the Schwann cells must provide support to the neurons and, therefore, this cell-to-cell interaction is a key feature that must be recreated to construct more representative *in vitro* models. With this method, it is possible to understand the effect of supplementation in the treatment of lesions to peripheral nerves, seeking to increase the regeneration rate of the neurites that extend through the lesion site, where they are supported and guided by the Schwann cells. It was therefore planned to recreate a culture model that recreates the neuron glial 3D interaction in a reliable and reproducible manner, as reported in the literature.

This *in vitro* growth of neurites imitates the key features of the physiology of the distal peripheral nerve during a repair following damage to the peripheral nerve. Therefore, the purpose of this second part of the analysis is to examine any direct effects, following intestinal passage, on the peripheral nerve, by analysing survival, intended as mitochondrial activity (viability) and the oxidative state, fundamental for the reduction of chronic pain. Furthermore, the main pathways involved in the processes of protection of the myelin sheath and formation of neurites, such as, for example, NRG1, MPZ, p75 and ERB3, will be analysed.

For this purpose, the damage to the peripheral nerves will be mimicked using "glial growth factor" (GGF), a consolidated model in literature, to simulate the lesions to the peripheral nerves, since it is capable of reproducing a robust demyelination.

In step 2, the product metabolised by the intestinal cells was placed directly in contact with EngNT. This technique was implemented by simulating damage to the peripheral nerve and demyelination with GGF and analysing:
- Cell viability assays with treatment with individual active ingredients and possible combination thereof
- Analysis of ROS Production
- Analysis of markers such as: NRG1, MPZ, p75 and ERB3

Preliminary results indicate the efficacy of the mixture and the composition containing said mixture.

### Results

### Results of step 1

### Viability

At any time point of the experiment, the MIX is demonstrated to be always synergistic in improving intestinal cell viability, compared to the active ingredients of which it is formed (Table 1).

The MIX is demonstrated to be statistically superior compared to the combination without lipoic acid and to the XINPROX formula (p<0.05 at all time points) (Figures 1 A and 1 B).

**Table 1**

| Cell viability | BPF | TRP | POLL | QUE | SUM | MIX |
|---|---|---|---|---|---|---|
| 1h | 6.46 | 2.74 | 2.04 | 1.51 | 12.74 | 24.14 |
| 2h | 14.02 | 3.57 | 2.90 | 2.36 | 22.86 | 30.96 |
| 3h | 9.11 | 6.38 | 4.56 | 3.25 | 23.30 | 40.18 |
| 4h | 6.73 | 2.52 | 1.77 | 1.25 | 12.27 | 37.01 |
| 5h | 1.49 | 0.17 | 0.04 | -0.07 | 1.63 | 33.50 |
| 6h | 1.35 | -1.44 | -1.12 | -0.87 | -2.07 | 21.49 |

### Integrity of the intestinal barrier

The active ingredients administered singly have a relatively low absorption percentage. In contrast, the mix of all the active ingredients has a very high absorption percentage. If the result is normalised with respect to the control, i.e. by subtracting the control reference from the TEER values of each experimental group, the synergy of the Mix with respect to the single ingredients is demonstrated (Table 2).

At any time point of the experiment, the mix is demonstrated always to be synergistic. The mix is demonstrated to be statistically superior p<0.05 compared to the other two mixtures (Figures 1 A and 1 B).

**Table 2**

| TEER | BPF | TRP | POLL | QUE | SUM | MIX |
|---|---|---|---|---|---|---|
| 1h | 7.20 | 8.90 | 12.30 | 5.37 | 33.77 | 52.82 |
| 2h | 9.60 | 10.30 | 14.10 | 7.20 | 41.20 | 65.82 |
| 3h | 7.80 | 10.70 | 14.90 | 8.69 | 42.09 | 79.40 |
| 4h | 5.80 | 7.38 | 11.00 | 4.87 | 29.05 | 73.08 |
| 5h | 4.80 | 6.32 | 10.40 | 3.38 | 24.90 | 68.49 |
| 6h | 5.00 | 6.10 | 10.20 | 3.24 | 24.54 | 50.50 |

### Tight junction activity

### Claudin

Claudin contributes to maintaining the structure of the tight junctions.

The active ingredients administered singly have little influence on the expression of claudin, in contrast, the action of the Mix is synergistic compared with the individual components (Figures 3 A and 3 B and Table 3). The mix is demonstrated to be statistically superior p<0.05 compared to the other two mixtures.

The MIX is synergistic compared with the active ingredients.

**Table 3**

| | BPF | TRP | POLL | QUE | SUM | MIX |
|---|---|---|---|---|---|---|
| Claudin | 11.356 | 5.472 | 5.216 | 4.986 | 27.03 | 30.06818 |

### Zonula occludens

Zonula occludens is the protein that mediates adhesion of the tight junction.

The individual active ingredients contribute minimally to its expression, in contrast, the Mix demonstrates its synergistic efficacy (Figures 4 A and 4 B and Table 4). The mix is demonstrated to be statistically superior p<0.05 compared to the other two mixtures. The MIX is synergistic compared with the active ingredients.

**Table 4**

| | BPF | TRP | POLL | QUE | SUM | MIX |
|---|---|---|---|---|---|---|
| ZO-1 | 6.976 | 3.878 | 3.682 | 3.622 | 18.158 | 24.25693277 |

### Absorption through the intestinal barrier

The individual active ingredients have a relatively low absorption profile. In contrast, when administered all together, they demonstrate an absorption synergy in the Mix (Figures 5 A and 5 B and Table 5).

At any time point of the experiment, the mix is demonstrated always to be synergistic.

**Table 5**

| Value of absorbance | BPF | TRP | POLL | QUE | SUM | MIX |
|---|---|---|---|---|---|---|
| 1h | 7.61 | 2.68 | 2.59 | 2.51 | 15.39 | 22.646 |
| 2h | 10.27 | 4.90 | 4.72 | 4.45 | 24.34 | 29.998 |
| 3h | 4.20 | 6.12 | 5.89 | 5.64 | 21.85 | 31.646 |
| 4h | 2.78 | 1.81 | 1.75 | 1.77 | 8.11 | 24.21 |
| 5h | 2.32 | 1.65 | 1.59 | 1.53 | 7.09 | 18.674 |
| 6h | 1.55 | 1.23 | 1.17 | 1.12 | 5.07 | 16.988 |

### Results Step 2

### Viability

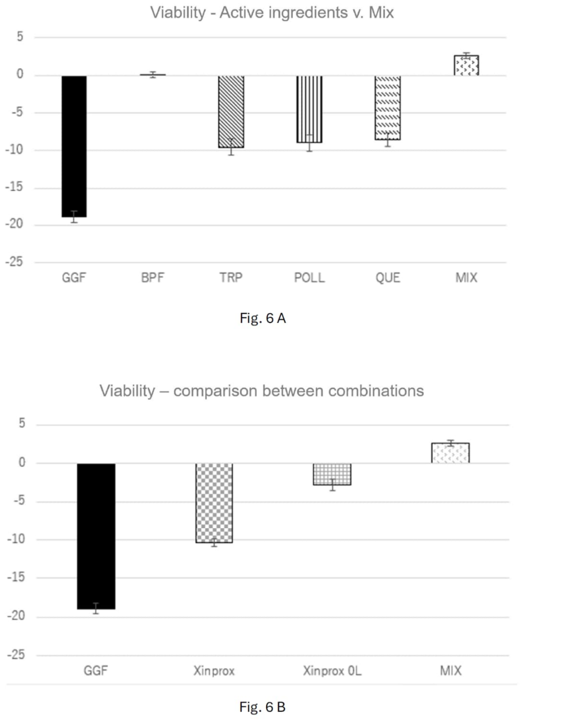

It was observed that only the bergamot is capable of completely eliminating the damage caused by GGF. Whereas the other active components are able to counter cell mortality only partially. The Mix is capable, with its synergistic action, of inverting the viability index, taking it to a positive value (Figures 6 A and 6 B and Table 6). The Mix is synergistic compared with the active ingredients. The mix is demonstrated to be statistically superior p<0.05 compared to the other two mixtures.

**Table 6**

| BPF | TRP | POLL | QUE | SUM | MIX |
|---|---|---|---|---|---|
| 0.0566 | -9.54 | -8.986 | -8.61 | -27.0794 | 2.647128 |

### Neuroprotection markers

The applicant has performed analysis of the level of specific markers involved in the processes of protection of the myelin sheath and formation of neurites.

### p75

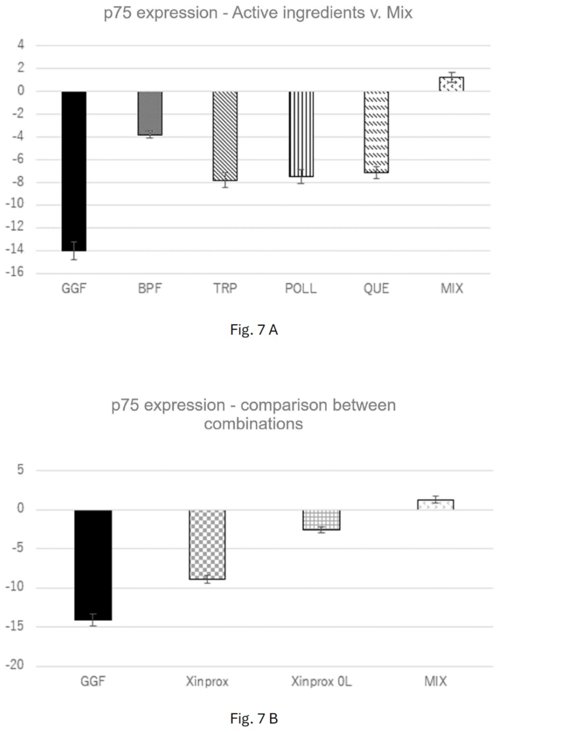

P75 is an important receptor for neurotrophins, involved in the plasticity and apoptosis processes. The individual active ingredients are only partially capable of countering the neuronal degeneration caused by the damage induced by GGF, whereas only the Mix is capable of inverting the trend and returning the p75 expression to levels comparable to the healthy control (Figures 7 A and 7 B and Table 7). The Mix is synergistic compared with the active ingredients. The mix is demonstrated to be statistically superior p<0.05 compared to the other two mixtures.

**Table 7**

| BPF | TRP | POLL | QUE | SUM | MIX |
|---|---|---|---|---|---|
| -3.78 | -7.808 | -7.488 | -7.106 | -26.182 | 1.24832 |

### MPZ

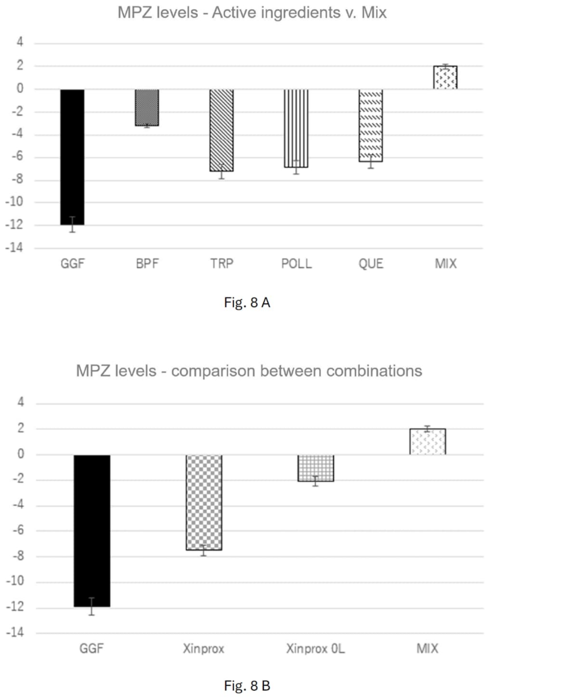

The previous result is also reconfirmed with the expression of MPZ, a structural protein of the myelin sheath. The individual active ingredients are only partially capable of countering the neuronal degeneration caused by the damage induced by GGF, whereas only the Mix is capable of inverting the trend and returning the MPZ expression to levels comparable to the healthy control (Figures 8 A and 8 B and Table 8). The Mix is synergistic compared with the active ingredients. The mix is demonstrated to be statistically superior p<0.05 compared to the other two mixtures.

**Table 8**

| BPF | TRP | POLL | QUE | SUM | MIX |
|---|---|---|---|---|---|
| -3.168 | -7.228 | -6.88 | -6.4 | -23.676 | 2.004 |

### NRG1

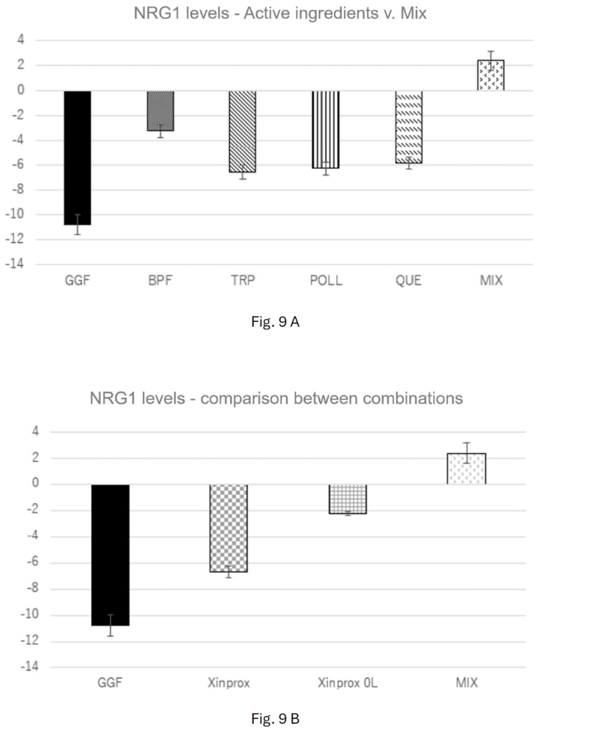

The role of Schwann cells was studied by analysing the modulation of neuropathic pain *in vitro.* The condition of peripheral neurodegeneration negatively influenced the expression of NRG1 following treatment with GGF. All the active ingredients administered singly reduced the damage, confirming their positive role in countering the demyelination process, but none of them was able to re-establish a physiological condition. The Mix demonstrated its synergistic action, inverting the neurodegenerative process and re-establishing a NRG1 value comparable to the healthy control (Figures 9 A and 9 B and Table 9). The Mix is synergistic compared with the active ingredients. The mix is demonstrated to be statistically superior p<0.05 compared to the other two mixtures.

**Table 9**

| Bergamot 100% | L-tryptophan | Graminex | Quercetin | SUM | Mix 0%L - 100%B |
|---|---|---|---|---|---|
| -3.211 | -6.518 | -6.256 | -5.846 | -21.831 | 2.404 |

### ERB3

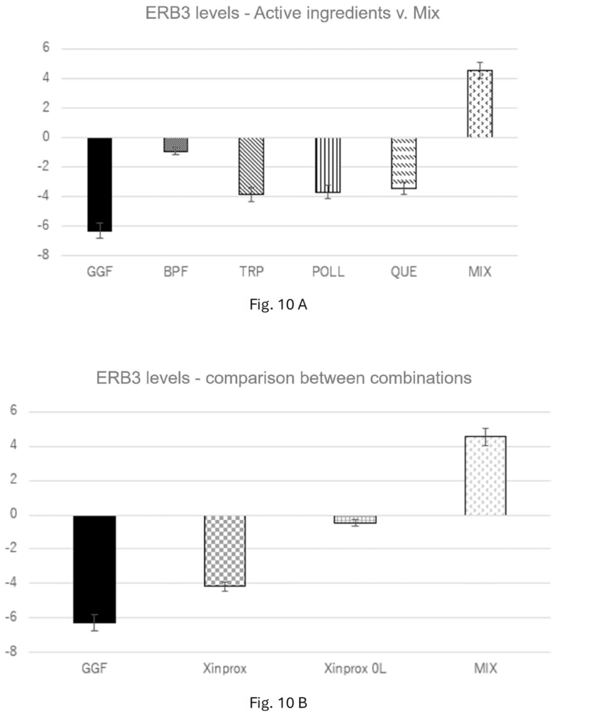

The role of Schwann cells was studied by analysing the modulation of neuropathic pain *in vitro.* The condition of peripheral neurodegeneration negatively influenced the levels of ERB3. All the active ingredients being studied reduced the damage, confirming their positive role in countering the demyelination process; however, none of them was able significantly to promote the expression of this marker. In contrast, the Mix, demonstrating its synergistic action, shows a distinctly higher level of expression of ERB3 (Figures 10 A and 10 B and Table 10). The Mix is synergistic compared with the active ingredients. The mix is demonstrated to be statistically superior p<0.05 compared to the other two mixtures.

**Table 10**

| GGF | BPF | TRP | POLL | SUM | MIX |
|---|---|---|---|---|---|
| -0.928 | -3.834 | -3.678 | -3.44 | -11.88 | 4.5504 |

### Conclusions

The data obtained demonstrates that:
- at intestinal level, the individual active ingredients and the mix are not toxic, demonstrating a high safety profile. Furthermore, the active ingredients, when mixed so as to obtain the mixture according to the invention, demonstrate a distinctly higher absorption, essential for reaching the neuron;
- in most of the markers considered in the step 2 studies on neuroprotection, the mixture was demonstrated to be much more active, at synergistic level, in restoring values comparable to the healthy control;
- the mixture according to the invention (MIX) is capable of acting on neuropathic pain in the pelvic area, attenuating the neuro-inflammation that characterises prostate diseases to a greater extent than the other mixtures tested;
- the role in attenuating neuro-inflammation played by the bergamot extract and the flavonoids it contains is considerable (see the comparison between the "Xinprox 0L" mixture and the "MIX" in Figures 7B -10B).

## Claims

1. A mixture comprising or, alternatively, consisting of:
a) a *Secale cereale* (L.) pollen extract; and
b) a quercetin, and
c) a bergamot extract, and
d) a tryptophan and/or a saffron extract.

2. The mixture according to claim 1, wherein said (a) rye *(Secale cereale* L.) pollen extract can comprise:
- a water-soluble pollen extract, hydrophilic fraction, preferably in an amount by weight comprised from 10% to 40%, more preferably comprised from 20% to 30%, e.g. 25%; and/or
- a fat-soluble pollen extract, lipophilic fraction, preferably in an amount by weight comprised from 0.1% to 5%, more preferably comprised from 1% to 3%, e.g. 1.5%; and, optionally,
- excipients in an amount by weight comprised from 55% to 89.9% with respect to the total weight of the pollen extract.

3. The mixture according to claim 1 or 2, wherein said (a) rye *(Secale cereale* L.) pollen extract can preferably be a rye *(Secale cereale* L.) pollen extract which can further also comprise a maize *(Zea mays* L.) extract and/or a timothy *(Phleum pratense* L.) extract.

4. The mixture according to any one of the preceding claims, wherein said (a) rye *(Secale cereale* L.) pollen extract comprises a hydrophilic fraction and a lipophilic fraction, in a ratio by weight comprised from 40:1 to 5:1, preferably comprised from 30:1 to 10:1, more preferably comprised from 20:1 to 15:1, by weight.

5. The mixture according to any one of the preceding claims, wherein said (c) bergamot extract comprises an amount of total flavonoids comprised from 10% to 60%, preferably comprised from 20% to 50%, more preferably from 30% to 40% by weight, with respect to the total weight of the extract.

6. The mixture according to any one of the preceding claims, wherein said total flavonoids present in said (c) bergamot extract comprise at least one flavonoid selected from the group comprising or, alternatively, consisting of naringin, neohesperidin, neoeriocitrin, melitidin and brutieridin; preferably, said flavonoids are naringin, neohesperidin, neoeriocitrin; more preferably, said flavonoids are naringin, neohesperidin, neoeriocitrin, melitidin and brutieridin.

7. The mixture according to any one of the preceding claims, wherein said mixture comprises:
- from 5% to 40% of naringin, preferably comprised from 20% to 30%, more preferably comprised from 28% to 29% by weight, with respect to the total weight of the flavonoids;
- from 5% to 40% of neohesperidin, preferably comprised from 20% to 35%, more preferably comprised from 25% to 30% by weight, with respect to the total weight of the flavonoids;
- from 5% to 40% of neoeriocitrin, preferably comprised from 20% to 30%, more preferably comprised from 25% to 26% by weight, with respect to the total weight of the flavonoids;
- from 1% to 10% of melitidin, preferably comprised from 3% to 7%, more preferably comprised from 4% to 5% by weight, with respect to the total weight of the flavonoids;
- from 5% to 20% of brutieridin, preferably comprised from 10% to 15%, more preferably comprised from 11% to 12% by weight, with respect to the total weight of the flavonoids.

8. The mixture according to any one of the preceding claims, wherein said mixture further comprises a saffron extract from *Crocus sativus (L.);* preferably, said extract comprises alpha-crocetin, crocin, picrocrocin and safranal, more preferably it comprises crocin and safranal.

9. The mixture according to claim 8, wherein said *Crocus sativus* extract is a dry extract containing crocin and/or safranal, the minimum content of safranal being ≥0.30% weight/weight and/or of crocin being ≥ 0.40% weight/weight, the safranal content preferably being in a range of 0.34% to 0.45% by weight, with respect to the total weight of the dry extract, and/or the crocin content preferably being in a range of 0.45% to 0.60% by weight, with respect to the total weight of the dry extract.

10. The mixture according to any one of the preceding claims, wherein component d) can comprise a tryptophan, in the form of L-tryptophan, and a saffron extract; preferably, said tryptophan and said saffron extract are present in a weight ratio comprised from 1:5 to 5:1, preferably comprised from 1:3 to 3:1, e.g. 1:1.

11. A composition comprising a mixture according to any one of the preceding claims and food or pharmaceutical grade excipients and/or additives.

12. The composition according to claim 11, wherein the daily dose unit of said composition comprises the following amounts:
(a) a *Secale cereale* pollen extract, preferably comprising a hydrophilic fraction:lipophilic fraction, in a ratio by weight comprised from 40:1 to 5:1, preferably comprised from 30:1 to 10:1, more preferably comprised from 20:1 to 15:1, e.g. equal to about 20:1 by weight, preferably in an amount by weight comprised from 50 mg to 2000 mg;
(b) a quercetin in an amount by weight comprised from 10 mg to 400 mg;
(c) a bergamot extract in an amount by weight comprised from 10 to 1000 mg; preferably, said bergamot extract has a content of total flavonoids neoeriocitrin, naringin, neohesperidin, melitidin, brutieridin comprised from 10% to 60%;
(d) a tryptophan, preferably in the form of L-tryptophan, in an amount comprised from 10 mg to 600 mg and, optionally, additives and excipients.

13. The composition according to claim 11 or 12, wherein the daily dose unit of said composition comprises the following amounts:
(a) a *Secale cereale* pollen extract, preferably comprising a hydrophilic fraction:lipophilic fraction, in a ratio by weight comprised from 40:1 to 5:1, preferably comprised from 30:1 to 10:1, more preferably comprised from 20:1 to 15:1, e.g. equal to about 20:1 by weight, in an amount comprised from 500 mg to 1000 mg;
(b) a quercetin in an amount by weight comprised from 100 mg to 200 mg;
(c)} a bergamot extract in an amount by weight comprised from 300 mg to 600 mg; preferably said bergamot extract has a content of total flavonoids neoeriocitrin, naringin, neohesperidin, melitidin, brutieridin comprised from 10% to 60%, preferably from 20% to 50%, more preferably from 30% to 40% by weight;
(d) a tryptophan in an amount by weight comprised from 150 mg to 300 mg and, optionally, additives and excipients.

14. The composition according to any one of claims 11-13, wherein said daily dose unit of said composition can be administered to an individual in need within a 24-hour interval with a single dose or divided into 2, 3 or 4 doses spaced apart at time intervals of 4 hours to 12 hours, depending on the type of dosage form and the individual's needs.

15. The composition according to any one of claims 11-14, wherein said composition is for oral or sublingual administration; preferably, said composition can be in solid form as a tablet, chewable tablet, effervescent tablet, capsule, soft capsule, lozenge, granules or powder, granules or powder to be dissolved in water or orally disintegrating; or in semi-solid form, such as soft gel; or in liquid form, such as a solution, suspension, dispersion, emulsion or syrup; more preferably, said composition is in solid form for oral use in the form of a tablet.

16. The mixture according to any one of claims 1-10 and the composition according to any one of claims 11-15, wherein said mixture and said composition are for use as a medicament.

17. The mixture or the composition for use according to claim 16, wherein said mixture and said composition are for use in a method of treatment, preventive and/or curative, of prostate pathologies and/or symptoms; preferably, wherein said prostate pathologies and/or symptoms are selected from non-bacterial prostatitis/chronic pelvic pain syndrome (CP/CPPS), benign prostate hyperplasia (BPH), and symptoms and/or disorders connected to said non-bacterial prostatitis/chronic pelvic pain syndrome (CP/CPPS) and benign prostate hyperplasia (BPH) in an individual having need, through the administration of a therapeutically effective amount of said mixture or composition to said individual.
